# EUROPÄISCHE PATENTSCHRIFT

(11) **EP 2 576 701 B1**
(45) Veröffentlichungstag und Bekanntmachung des Hinweises auf die Patenterteilung: **23.03.2016**
(21) Anmeldenummer: 11738602.9
(22) Anmeldetag: 03.06.2011
(51) Int. Cl.: C09B 67/00, A61K 9/00, A61K 47/06

(54) **ZUBEREITUNG ZUR VERWENDUNG IN DER AUGENHEILKUNDE UND NETZHAUTCHIRURGIE**
PREPARATION FOR USE IN OPHTHALMOLOGY AND RETINAL SURGERY
PRÉPARATION POUR UTILISATION DANS OPHTALMOLOGIE ET CHIRURGIE RÉTINIENNE

(30) Priorität: 02.06.2010 DE 102010022567
(43) Veröffentlichungstag der Anmeldung: 10.04.2013
(73) Patentinhaber: Fluoron GmbH, 89077 Ulm (DE)
(72) Erfinder: HAGEDORN, Nadine, 89134 Blaustein (DE); RIZZO, Stanislao, 55100 Lucca (IT); RODRIGUES, Eduardo, João Paulo Florianopolis SC (BR)
(74) Vertreter: Schiweck, Weinzierl & Koch
(86) Internationale Anmeldenummer: PCT/EP2011/002748
(87) Internationale Veröffentlichungsnummer: WO 2011/151079

(56) Entgegenhaltungen:
- WO-A2-03/079927
- DE-A1- 19 536 504
- DE-C1- 19 719 280
- US-A1- 2002 128 527
- US-B1- 6 262 126
- DATABASE WPI Week 200456 Thomson Scientific, London, GB; AN 2004-577935 XP002667540, & RU 2 232 001 C2 (MIKROKHIRURGIYA GLAZA SCI TECH COMPLEX) 10. Juli 2004 (2004-07-10)

## Beschreibung

### Gebiet der Erfindung

Die Erfindung betrifft eine farbstoffhaltige Zubereitung zur Verwendung in der Augenheilkunde und Netzhautchirurgie.

### Hintergrund der Erfindung

Das Auge von Wirbeltieren ist ein wichtiges und ebenso empfindliches Organ. Ist seine Funktionsfähigkeit eingeschränkt oder fehlt sie ganz, so sind Tiere in der freien Wildbahn oft nicht mehr lebensfähig. Wenngleich beim "Wirbeltier Mensch" die Beeinträchtigung der Sehfähigkeit nicht mehr lebensentscheidend ist, so bedeutet es doch ein großes Stück Lebensqualität und Selbständigkeit über gesunde Augen zu verfügen. Aufgrund dessen werden die Sehfähigkeit erhaltende Augenoperationen immer bedeutungsvoller.

Die Möglichkeiten zur Behandlung von Erkrankungen am Auge, insbesondere der Netzhaut, wie z.B. Netzhautablösung oder Netzhautveränderung, haben in den letzten Jahren stark zugenommen. Teil einer derartigen Behandlung ist zumeist die operative Entfernung des Glaskörpers (Vitrektomie). Zur Entfernung des Glaskörpers werden drei kleine Schnitte in der Sklera des Auges vorgenommen, die in die hintere Augenkammer reichen, in der sich der Glaskörper befindet. Durch die Einschnitte können dann entsprechende Operationsinstrumente in das Innere des Auges eingeführt werden. Während des Eingriffs wird das Operationsfeld gespült, in der Regel mit Kochsalzlösung, da diese kompatibel ist mit dem Wasser des Glaskörpers, so dass ein Austausch der beiden Flüssigkeiten problemlos erfolgen kann. Damit das Auge aufgrund des Druckverlustes beim Absaugen des die Stabilität bildenden Glaskörpers nicht die Form verliert und Sekundärschäden erleidet, wird an Stelle des Glaskörpers eine Ersatzflüssigkeit in den Hohlraum eingeleitet, die den Innendruck konstant hält. Alle im Augeninneren eingesetzten interoperativen Mittel, wie Infusionslösungen, Spüllösungen und solche interoperative Lösungen, die zur Stabilisierung der Netzhaut und Verhinderung deren Krümmung und Ablösung in das Auge eingeführt werden, müssen physiologisch verträglich sein, sich gut einführen und leicht wieder entfernen lassen.

WO 03/079927 schlägt die Verwendung von perfluorierten Alkanen als Austauschflüssigkeit und Tamponadepräparat bei chirurgischen Eingriffen am Auge vor, insbesondere zu operativen Eingriffen an der Netzhaut. Perfluorierte Alkane haben den Vorteil, sich aufgrund ihrer hohen Dichte, die zumeist in einem Bereich von 1,8 bis 2,0 g/cm³ liegt, besonders im unteren Teil des Glaskörpers anzusammeln und somit die Behandlung für den Chirurgen an der Netzhaut zu erleichtern. Sie füllen dabei den Glaskörperraum von der posterioren Netzhaut her auf. Perfluorierte Alkane zeichnen sich durch eine ausgesprochene Hydrophobie und Lipophobie aus, das heißt, sie sind weder öl- noch wasserlöslich und vermischen sich daher auch nicht mit Blut oder Körperflüssigkeiten, so dass sie ihre hohe Transparenz beibehalten und den Operationsort für den Operateur stets ungetrübt erscheinen lassen. Ebenso werden die Perfluoralkane nicht vom Körper aufgenommen und ferner auch nicht verstoffwechselt. Kein im menschlichen oder tierischen Körper vorhandenes Enzymsystem ist in der Lage, perfluorierte Alkane zu spalten und abzubauen. Perfluoralkane sind daher bevorzugte interoperative Flüssigkeiten.

In den Dokumenten DE -C1-19719280 sowie DE -A1-19536504 gelangen u.a. auch Gemische aus teil- sowie perfluorierten Alkanen zur Anwendung in der Augenheilkunde.

Die RU -C2- 2232001 (siehe dessen Zusammenfassung in der Datenbank der Thomson Scientific, London) beschreibt ein chirurgisches Verfahren zur Behandlung einer schweren Form der Netzhautablösung, bei dem es zum Einsatz des Farbstoffs Indocyaningrün und PFOS (Perfluoroktansulfonat) kommt.

Die Behandlung von komplizierten Netzhautablösungen gestaltet sich in der Regel wie folgt: Nach erfolgter partieller oder kompletter Vitrektomie wird die Netzhaut mittels schwerer Flüssigkeiten, d.h. Perfluorcarbone bzw. Perfluoralkane, wie z.B. Perfluoroctan oder Perfluordecalin, wieder angelegt. Aufgrund der niedrigen Viskosität kann mit Standard-Kanülen von 20 bis 23 G direkt über der optischen Papille instilliert werden. Durch ihre hohe Dichte sinken Perfluorcarbone auf die posteriore Netzhaut ab und füllen von unten langsam den Glaskörperraum auf. Diese hohe Dichte macht die Perfluorcarbone zu hervorragenden intraoperativen Instrumenten für die anstehenden Netzhautmanipulationen. Nach dem chirurgischen Eingriff und der Stabilisierung der Netzhaut werden Perfluoroctan bzw. Perfluordecalin aus dem Glaskörperraum wieder abgesaugt und durch traditionelle Gas- oder Flüssig-Endotamponaden ersetzt. Um den Erfolg des operativen Eingriffs nicht zu gefährden, ist es daher wichtig, die interoperativen Flüssigkeiten, wie die Perfluoralkane, nach erfolgter Operation vollständig aus dem Glaskörperraum wieder zu entfernen. Verbleiben dünne Filme oder kleine Tröpfchen von perfluoriertem Alkan auf der Netzhaut zurück, so kann dies zu unerwünschten Nebenwirkungen, wie Entzündungsreaktionen oder sekundären Sehbehinderungen führen. Aufgrund der hohen Transparenz der Perfluoralkane ist ein vollständiges Entfernen derselben vom Operationsort oft schwierig. Außerdem ist es aufgrund der guten Vermischbarkeit möglich, die Färbung der Zubereitung individuell für jeden Einzelfall einzustellen.

Aufgabe ist es daher, eine Zubereitung zur Verwendung in der Augenheilkunge, insbesondere der Netzhautchirurgie bereitzustellen, die den operativen Eingriff erleichtert und Sekundärschäden verhindert. Ferner ist es Aufgabe, eine Zubereitung bereitzustellen, die sicher in ihrer Anwendung ist, gezielt eingesetzt werden kann und ferner den Vorteil birgt, dass sie ohne den Blick auf das Operationsfeld zu trüben, visualisiert werden kann, so dass eine Überprüfung auf ihre An- oder Abwesenheit leicht durchgeführt werden kann.

Die Aufgabe wird gelöst durch eine Zubereitung, wie sie in Anspruch 1 definiert ist. Die Unteransprüche enthalten vorteilhafte Weiterbildungen.

Überraschend wurde gefunden, dass eine Zubereitung, die mindestens ein perfluoriertes Alkan, mindestens ein semifluoriertes Alkan und mindestens einen in dem semifluorierten Alkan gelösten Farbstoff enthält, wobei das semifluorierte Alkan ausgewählt ist aus Verbindungen der nachfolgenden Formel

R_{F}R_{H} oder R_{F}R_{H}R_{F}

wobei R_{F} eine lineare oder verzweigte Perfluoralkylgruppe mit 3 bis 20 Kohlenstoffatomen und RH eine lineare oder verzweigte, gesättigte Alkylgruppe mit 1 bis 20 Kohlenstoffatomen ist, wobei die Kohlenstoffkette insgesamt 4 bis 30, bevorzugt 8 bis 20 Kohlenstoffatome aufweist und wobei das Verhältnis des perfluorierten Alkans zu dem semifluorierten Alkan 95:5 bis 5:95 beträgt, sich als interoperative Flüssigkeit optimal eignet.

Die erfindungsgemäße Zubereitung ist leicht ohne großen technischen Aufwand herstellbar, wobei all ihre Komponenten gut erhältlich sind. Die Zubereitung zeichnet sich durch eine hohe physiologische Verträglichkeit und ein geringes Reizpotenzial aus und hat eine ausreichend geringe Toxizität, dass sie im empfindlichen Augeninneren bedenkenlos eingesetzt werden kann. Aufgrund der hohen strukturellen Kompatibilität von Perfluoralkan und Semifluoralkan können aus diesen beiden Komponenten über weite Bereiche Mischungen hergestellt werden, die sich durch Homogenität und Einphasigkeit auszeichnen. Dabei kann sowohl das Perfluoralkan wie auch das Semifluoralkan jeweils wiederum eine Mischung aus Perfluoralkanen bzw. Semifluoralkanen sein, Die Transparenz der Zubereitung wird nicht nachteilig beeinträchtigt, da sich Perfluoralkane und semifluorierte Alkane in jedem Verhältnis homogen miteinander mischen lassen und die Mischung der Zubereitung einphasig und transparent ist und überraschenderweise auch bei Zusatz eines erfindungsgemäßen Farbstoffs transparent bleibt.

Ferner ist durch die erfindungsgemäße Kombination von perfluoriertem Alkan mit semifluoriertem Alkan die Dichte der Zubereitung optimal einstellbar. Dies ist wesentlich, da die Dichte mancher Perfluoralkane als zu hoch angesehen wird, und bei deren Verwendung zu viel Druck auf die Netzhaut ausgeübt wird, die dann Schäden erleiden kann. Eine hohe Dichte der Zubereitung, also eine Dichte von größer als 1,33 g/cm³, vorzugsweise größer als 1,5 g/cm³ und insbesondere von größer als 1,7 g/cm³ ist erfindungsgemäß bevorzugt, da dies ein schnelles Absinken der Zubereitung auf die Netzhaut garantiert und die Stabilisierung und damit den operativen Eingriff beschleunigt. Andererseits ist eine Dichte in diesem Bereich auch nicht so hoch, dass die Netzhaut durch zu hohen Druck geschädigt wird. Die Dichte der Lösungen kann bei Raumtemperatur (20-25°C) z.B. mittels Schwingkörper-Methode ermittelt werden.

Perfluorierte Alkane können aufgrund ihrer physikalischen Eigenschaften nicht direkt gefärbt werden, erfindungsgemäß wird daher eine Zubereitung bereitgestellt, die eine Färbung der Perfluoralkane zulässt, indem mindestens ein perfluoriertes Alkan mit mindestens einem semifluorierten Alkan und einem darin gelösten Farbstoff kombiniert wird. Es entsteht eine gefärbte, einphasige Lösung. Dies ist sowohl auf die Löslichkeit der farbgebenden Substanz in dem semifluorierten Alkan wie auch die Löslichkeit des semifluorierten Alkans in dem Perfluoralkan zurückzuführen und ist für die erfindungsgemäße Zubereitung wesentlich.

Die Färbung der Zubereitung ist wichtig, um beim Ausspülen der besagten interoperativen Zubereitung überprüfen zu können, ob auch alle Reste der Zubereitung vollständig ausgeschwemmt wurden. Zurückbleibende Reste könnten hingegen zu Reizungen oder Entzündungen und in schlimmen Fällen zur Netzhautablösung führen. Die erfindungsgemäße Zubereitung ermöglicht aufgrund ihrer physikalischen Eigenschaften, und insbesondere ihrer chemischen, physiologischen wie biologischen Inertheit und ihrer Dichte damit eine optimale Stabilisierung der Netzhaut vor, während bzw. nach dem operativen Eingriff im Augeninneren, also insbesondere vor Einführung der Glaskörpertamponade. Sie ermöglicht gleichzeitig eine rückstandslose Entfernung derselben aus dem Augeninneren, wodurch Sekundärschäden nach der Operation vorgebeugt bzw. verhindert werden. Da die in der erfindungsgemäßen Zubereitung enthaltenen Komponenten über eine hohe chemische wie auch physikalische Stabilität verfügen, ist die Zubereitung auch gut sterilisierbar, was wichtig ist, um nicht durch Keime Schädigungen am Augeninneren zu verursachen.

Unter einer interoperativen Zubereitung im Sinne der Erfindung wird eine Flüssigkeit verstanden, die der Stabilisierung der Netzhaut vor, während oder nach einem operativen Eingriff dient, also zum Beispiel nach Absaugung des Glaskörpers zur Verhinderung der Krümmung oder des vollständigen Ablösens der Netzhaut vor Einführung der Glaskörpertamponade in den Glaskörperraum des Auges.

Semifluorierte Alkane sind in der Ophthalmologie bekannt und werden z.B. in EP 0 859 751 beschrieben, auf deren Inhalt hier ausdrücklich Bezug genommen werden soll. Unter dem Begriff semifluorierte Alkane werden allgemein Verbindungen verstanden, die einen Block eines gesättigten linearen oder verzweigten Alkans und ein oder zwei Blöcke eines perfluorierten Alkans aufweisen. Die erfindungsgemäß verwendeten semifluorierten Alkane weisen somit eine blockartige Struktur auf, in der Blöcke von perfluorierten Alkylresten in Nachbarschaft zu nichtfluorierten gesättigten Alkylresten vorhanden sind. Die linearen semifluorierten Alkane haben daher folgende Diblock- bzw. Triblockstruktur:

F(CF₂)ₘ(CH₂)ₙH oder F(CF₂)ₘ(CH₂)ₙ(CF₂)ₒF

wobei m und o jeweils ganze Zahlen von 3 bis 20, vorzugsweise von 3 bis 8 sind und n eine ganze Zahl von 1 bis 20, vorzugsweise von 4 bis 8 ist, wobei die Gesamtlänge der Kohlenstoffkette 4 bis 30, bevorzugt 8 bis 20 Kohlenstoffatome beträgt. Semifluorierte Alkane werden häufig abgekürzt mit der Anzahl der F-tragenden und H-tragenden C-Atome benannt, z.B. F₆H₆ für C₆F₁₃C₆H₁₃ oder Perfluorhexylhexan.

Verzweigte semifluorierte Alkane können neben oben genannten Strukturbestandteilen ferner im perfluorierten Anteil zusätzliche FCX-Einheiten und im Alkanteil zusätzliche HCY-Einheiten aufweisen, wobei X wiederum ein Perfluoralkylrest ist und bevorzugt 1 bis 8 und besonders bevorzugt 2 bis 4 Kohlenstoffatome aufweist und wobei Y ein Alkylrest mit vorzugsweise 1 bis 8 und besonders bevorzugt 2 bis 4 Kohlenstoffatomen ist. Ebenso können die randständigen F₃C- und H₃C-Gruppen durch FCX₂ oder F₂CX bzw. HCY₂-Gruppen oder H₂CY-Gruppen ersetzt sein, wobei X und Y eine Struktur wie bereits oben dargestellt aufweisen. Wie für die linearen semifluorierten Alkane, gilt auch für die verzweigten semifluorierten Alkane, dass die Summe an Kohlenstoffatomen vorzugsweise 20 nicht überschreitet. Durch derartige Modifikationen können individuelle semifluorierte Alkane hergestellt werden, die je nach Einsatzgebiet variiert werden können. Durch den nichtsubstituierten Alkylanteil sind die semifluorierten Alkane durch eine geringe bis mittlere Lipophilie ausgezeichnet, das heißt, dass sie in der Lage sind, sich in geeigneten lipophilen Lösungsmitteln zu lösen bzw. lipophile Komponenten in sich zu lösen. Dies ist wesentlich für die vorliegende Erfindung, da es nur aufgrund dieser Lipophilie möglich ist, durch Lösung einer ebenfalls lipophilen farbgebenden Substanz der erfindungsgemäßen Zubereitung Farbe zu verleihen.

Die semifluorierten Alkane sind an sich transparente Flüssigkeiten, die physiologisch kompatibel, im Handel erhältlich und nicht toxisch sind. Ferner zeichnen sie sich durch ein niedriges Reizpotenzial aus. Sie sind inert, reagieren also nicht mit am Operationsort vorhandenen Körperflüssigkeiten und Zellen und werden auch nicht verstoffwechselt und können daher optimal als Komponente in interoperativen Flüssigkeiten in der Netzhautchirurgie eingesetzt werden.

Perfluorierte Alkane im Sinne der Erfindung sind gesättigte lineare, verzweigte oder cyclische Alkane mit C-F-Bindungen. Geeignete Perfluoralkane sind dem Fachmann z.B. aus WO 03/079927 bekannt und umfassen vorzugsweise solche mit 4 bis 20 und besonders bevorzugt 4 bis 12 Kohlenstoffatomen, z.B. C₄F₁₀, C₅F₁₂, C₆F₁₄, C₇F₁₆, C₈F₁₈, C₉F₂₀, C₁₀F₁₈ oder C₁₀F₂₂, C₁₁F₂₄ oder C₁₂F₂₆ oder Mischungen davon. Besonders bevorzugt sind Perfluordecalin und Perfluoroktan. Auch Mischungen von Isomeren und/oder Mischungen von Perfluoralkanen unterschiedlicher Kettenlänge sind geeignet, z.B. eine Mischung von Perfluorheptan, Perfluoroktan und Perfluornonan. Erfindungsgemäß geeignet sind flüssige Perfluoralkane, die einen hohen Brechungsindex und eine hohe Transparenz aufweisen und sich ferner durch eine Viskosität auszeichnen, die sie leicht in den Glaskörperraum einführbar und daraus wieder entfernbar macht. Dies ist wichtig, um beim operativen Eingriff möglichst keine Schäden z.B. an der Sklera des Auges zu hinterlassen, durch die die interoperative Flüssigkeit eingeführt werden soll, was vorzugsweise mittels einer sehr dünnen Nadel von 20 bis 23 oder sogar 25 Gauge erfolgt.

Perfluoralkane werden in der Netzhautchirurgie aufgrund ihrer hohen Dichte und Inertheit eingesetzt. Allerdings können Perfluoralkane aufgrund ihrer ausgesprochenen Hydrophobie und Lipophobie nicht mit anderen nützlichen Substanzen vermischt werden. Ein hoher Anteil von perfluoriertem Alkan ist, wie bereits ausgeführt, erforderlich, um die Dichte der Zubereitung auf einen Wert zu bringen, so dass die Zubereitung vollständig und ohne erhebliche zeitliche Verzögerung auf die Netzhaut absinkt und die Netzhaut damit optimal stabilisiert. Um diesen positiven Effekt zu erzielen, ist es erforderlich, dass das Mischungsverhältnis, bezogen auf Volumen, 95:5 bis 5:95 Perfluoralkan zu semifluoriertem Alkan beträgt. Je höher der Anteil an Perfluoralkan, desto höher ist die Dichte der Gesamtzubereitung und desto schneller und vollständiger sinkt die Zubereitung auf die Netzhaut im Augeninneren und stabilisiert diese. Das Mischungsverhältnis wird also so gewählt, dass die vorbenannten positiven Eigenschaften der Gesamtzusammensetzung erzielt werden. Ein gewisser Anteil an semifluoriertem Alkan ist allerdings notwendig, um die Farbigkeit der erfindungsgemäßen Zubereitung bereitzustellen. Durch einfache Routineversuche kann der Fachmann das optimale Verhältnis in Abhängigkeit der verwendeten Komponenten herausfinden.

Perfluoralkane und semifluorierte Alkane (SFA) mischen sich in jedem Verhältnis, die Auswahl der jeweils zu mischenden Verbindungen ist daher unkritisch, solange sie bei Raumtemperatur (d.h. etwa 25°C) und Körpertemperatur (d.h. etwa 37°C) flüssig sind. Bevorzugte Kombinationen sind Perfluoralkane mit 8 oder 10 Kohlenstoffatomen mit semifluorierten Alkanen mit 8 bis 16 Kohlenstoffatomen. Vorteilhafte Mischungen werden beispielsweise erhalten aus Perfluordekalin und/oder Perfluoroktan mit C₄F₉C₅H₁₁, C₆F₁₃C₆H₁₃ und/oder C₆F₁₃C₈H₁₇.

Vorzugsweise beträgt der Anteil an semifluoriertem Alkan 5 bis 60 Vol.-% bezogen auf das Gesamtvoluumen der erfindungsgemäßen Zubereitung, bevorzugt 7 bis 50 Vol.-%, bevorzugter 10 bis 40 Vol.-% und besonders bevorzugt 10 bis 30 Vol.-%. Liegt der Anteil an semifluoriertem Alkan in dem angegebenen Bereich, so kann eine ausreichende Menge an farbgebender Substanz darin gelöst werden, so dass die erfindungsgemäße Zubereitung insgesamt eine visuell gut sichtbare Farbintensität aufweist, die am Applikationsort gut wahrgenommen werden kann. Ein Anteil von weniger als 5 Vol.-% reicht in der Regel nicht aus, um eine ausreichende Färbung zu erzielen. Bei Anteilen von mehr als 60 Vol.-% an semifluoriertem Alkan, sinkt die Dichte der Gesamtzubereitung auf einen Wert, bei dem die Zubereitung die Netzhaut möglicherweise nicht mehr ausreichend an Ort und Stelle stabilisieren kann. Die Zubereitung schwimmt im Glaskörperraum und es besteht die Gefahr, dass sie beim Spülen oberhalb des Operationsraumes einfach mit ausgeschwemmt wird.

Ein weiterer erfindungswesentlicher Bestandteil ist ein Farbstoff als farbgebende Substanz, der sich in dem semifluorierten Alkan löst. Unter Farbstoffen werden allgemein Substanzen verstanden, die sich im Gegensatz zu Pigmenten in Lösemitteln lösen. Weiterhin werden unter Farbstoffen bzw. farbgebenden Substanzen im Sinne der Erfindung solche Substanzen verstanden, die Licht im sichtbaren Wellenlängenbereich, also von etwa 350 bis 750 nm, reflektieren und/oder durch Anregung mittels Energie z.B. in Form von Wärme oder Licht zur Reflexion von Licht einer Wellenlänge im sichtbaren Wellenlängenbereich angeregt werden können.

Für die vorliegende Erfindung geeignet sind Lösemittelfarbstoffe und Fettfarbstoffe, soweit sie in den erfindungsgemäß verwendeten semifluorierten Alkanen löslich sind und physiologisch kompatibel sind. Als "Lösemittelfarbstoff" wird eine Substanz bezeichnet, die über eine Eigenfarbe im sichtbaren Spektralbereich des Lichtes verfügt und in aprotischen, lipophilen Lösemitteln löslich ist. Sie zeichnet sich zumeist durch eine ebenfalls lipophile Grundstruktur aus und ist deshalb auch in den erfindungsgemäß verwendeten semifluorierten Alkanen gut löslich. Als Fettfarbstoffe werden üblicherweise fett- und öllösliche Farbstoffe bezeichnet, d.h. Farbstoffe, die sich in lipophilen Lösungsmitteln lösen. Beispiele für einen Fettfarbstoff sind Farbstoffe der Sudanklasse, wie z.B. Sudan I, Sudan II, Sudan III und Sudan IV.

Der erfindungsgemäß verwendete Farbstoff kann ein Fluoreszenzfarbstoff sein, also ein Farbstoff, der nach Einwirkung von Energie, insbesondere in Form von Wärme oder Licht - im allgemeinen Strahlung, zur Aussendung von Licht im sichtbaren Spektralbereich angeregt wird und dann fluoresziert. Auch bei den Fluoreszenzfarbstoffen ist eine ausreichende Löslichkeit in dem semifluorierten Alkan wesentlich, da ansonsten keine Färbung der erfindungsgemäßen Gesamtzubereitung erzielt werden kann.

Erfindungsgemäß kommen solche Farbstoffe in Betracht, die sich in semifluorierten Alkanen in einem solchen Maß lösen, dass die Lösung sichtbar gefärbt ist. Unter "sichtbar gefärbt" wird dabei verstanden, dass mit unbewaffnetem Auge erkennbar ist, dass die Lösung eine Farbe aufweist, z.B. wenn sie vor weißem Hintergrund betrachtet wird. Unter "lösen" wird verstanden, dass sich zumindest so viel von der farbgebenden Substanz bei Raumtemperatur in dem semifluorierten Alkan homogen löst, dass eine ausreichende Färbung der Zubereitung insgesamt erzielt wird und keine Bildung einer Zweitphase auftritt. Praktisch wird so viel farbgebende Substanz in semifluoriertem Alkan gelöst, bis nach Vermengung mit der gewünschten Menge an perfluoriertem Alkan eine ausreichende Farbtiefe der Gesamtzubereitung bei der Temperatur am Operationsort (in der Regel etwa 35 bis 37°C) erzielt ist. Da bezüglich der Vermischbarkeit keine Grenzen gesetzt sind, kann das Verhältnis jeweils optimal eingestellt werden, d.h. die Farbtiefe kann individuell an Anwendungsort, Operateur, Lichtbedingungen etc. angepasst werden. Die Farbtiefe kann verändert werden, indem der Anteil an Farbstoff, der in dem semifluorierten Alkan gelöst ist, variiert wird und/oder indem der Anteil an gefärbtem semifluorierten Alkan in der Gesamtzubereitung variiert wird. Etwaige ungelöste Anteile an farbgebender Substanz müssen vor der Weiterverarbeitung der erfindungsgemäßen Zubereitung abfiltriert werden. Die Farbtiefe kann kann leicht mit dem Fachmann bekannten Mitteln, z.B. mittels UV-VIS-Spektroskopie oder anderen geeigneten photometrischen Verfahren bestimmt werden.

Prinzipiell kann jeder in dem semifluorierten Alkan lösliche Farbstoff verwendet werden, wenn er in der angewendeten Konzentration nicht toxisch ist und über eine ausreichende Biokompatibilität, physiologische wie chemische Inertheit und niedriges Reizpotenzial verfügt, so dass das Gewebe beim operativen Eingriff nicht beschädigt wird. Die Löslichkeit der erfindungsgemäß verwendeten farbgebenden Substanz ist bei der Temperatur, die am Operationsort herrscht so hoch, dass keine Zweitphasenbildung, etwa durch Ausfällen der farbgebenden Substanz, auftritt.

Bevorzugt wird ein solcher Farbstoff verwendet, der in möglichst geringer Menge noch eine sichtbare Färbung liefert. Besonders geeignete, aber nicht darauf beschränkte Beispiele von Farbstoffen im Sinne der Erfindung sind Farbstoffe, ausgewählt aus der Gruppe bestehend aus: Solvent Blue 36, Solvent Blue 35, Solvent Green 5, Solvent Violet 13, Solvent Blue 8, Solvent Blue 18, Solvent Blue 63, Solvent Green 3, Solvent Green 7, Solvent Violet 10, Solvent Violet 12, Solvent Violet 26, Solvent Red 27, Solvent Yellow 56, Solvent Green 3, Solvent Yellow 33, Solvent Red 19, Solvent Red 1, Solvent Yellow 16, wobei Solvent Blue 36, Solvent Blue 35, Solvent Green 5, Solvent Violet 13, sowie Sudan I bis IV aufgrund ihrer ausgesprochen guten physiologischen Verträglichkeit und guten Löseeigenschaften in semifluorierten Alkanen bevorzugt sind. Auch Mischungen von Farbstoffen können verwendet werden, z.B. wenn ein spezieller Farbton erzielt werden soll. Bevorzugt wird für die Zubereitung jeweils nur ein Farbstoff verwendet.

Der erfindungsgemäße Farbstoff wird in einer Menge verwendet, die ausreichend färbt. Die jeweils geeignete Menge hängt ab von dem jeweils verwendeten Farbstoff und kann vom Fachmann mit Routineversuchen einfach bestimmt werden. In der Regel reicht, insbesondere bei den oben als bevorzugt genannten Farbstoffen, eine Menge von weniger als 0,08 g/L aus, um eine ausreichend sichtbare Färbung der Zubereitung zu erhalten. Bevorzugt wird der Farbstoff in einer Menge von 0,08 bis 0,001 g/L, bevorzugter 0,07 bis 0,003 g/L, beispielsweise 0,06 g/L (=0,006%) bis 0,006 g/L (=0,0006%) eingesetzt. Dabei beziehen sich die angegebenen Mengen jeweils auf das Gesamtgewicht der fertigen Zubereitung.

Die erfindungsgemäße Zubereitung kann neben den zuvor beschriebenen Komponenten zusätzlich arzneimittelwirksame Bestandteile wie antibiotisch wirkende Substanzen, z.B. Cyclosporin, Glucocorticoide wie Triamcinolon, Dexamethason oder Wirkstoffe wie Indomethacin, Colchizin, Heparin und Zytostatika enthalten. Zudem können der Mischung fettlösliche Antioxidantien wie Lutein, Zeaxanthin, Vitamin E beigesetzt werden.

Die Herstellung der erfindungsgemäßen Zubereitung kann dadurch erfolgen, dass die farbgebende Substanz in der vorgesehenen Menge an semifluorierten Alkan unter Zuhilfenahme eines geeigneten Rührorgans homogen vermischt und im Anschluss das Perfluoralkan zugegeben wird. Die Zubereitung wird über Nacht bei Raumtemperatur in einem geschlossenen Gefäß aufbewahrt und vor Weiterverarbeitung filtriert. Die erfindungsgemäße Zubereitung kann für jeden Eingriff individuell gemischt werden. Bevorzugt wird eine vorgemischte Lösung bereitgestellt, besonders bevorzugt in Form einer Infusionslösung.

Die erfindungsgemäße Zubereitung eignet sich ideal zur Verwendung in der Augenheilkunde, insbesondere in der Netzhautchirurgie und ist besonders geeignet bei Vitrektomie bzw. für chirurgische Eingriffe im Zusammenhang mit Netzhautablösung.

Gegenstand der Erfindung ist daher auch die Verwendung der erfindungsgemäßen Zubereitung in der Augenheilkunde, in der Netzhautchirurgie, insbesondere für Vitrektomie oder chirurgische Eingriffe im Zusammenhang mit Netzhautablösung.

### Beispiele

Soweit nicht anders angegeben beziehen sich die Mengenangaben für den Farbstoff auf Gewichtsteile bezogen auf das Gesamtvolumen der Zubereitung. Die Mengenangaben für die Lösungsmittel sind Vol.% und beziehen sich auf das Gesamtvolumen. Für die Färbung wurden Grundlösungen an Farbstoff angesetzt, die jeweils einen spezifischen Farbstoff in einer Konzentration von 0,06 g/L in semifluoriertem Alkan enthielten. Von diesen Grundlösungen wurden dann für die Herstellung der Beispielslösungen jeweils die in den Tabellen angegebenen Volumenanteile entnommen und mit Perfluoralkan in der angegebenen Menge vermischt.

### Beispiel 1

| Farbstoff | Gehalt Semifluoriertes Alkan C₆F₁₃-C₈H₁₇ | Gehalt Perfluoriertes Alkan Perfluoroktan | Konzentration Farbstoff in g/L | Dichte [g/cm³] | Farbintensität |
|---|---|---|---|---|---|
| Solvent Blue 36 | 10 Vol.-% | 90 Vol.-.% | 0,006 | 1,71 | zart |
| Solvent Blue 36 | 20 Vol.-% | 80 Vol.-% | 0,012 | 1,66 | leicht |
| Solvent Blue 36 | 30 Vol.-% | 70 Vol.-% | 0,018 | 1,62 | mittel |
| Solvent Blue 36 | 60 Vol.-% | 40 Vol.-% | 0,036 | 1,58 | stark |

### Farbstoff

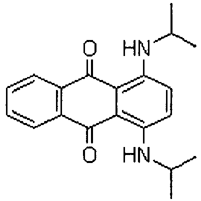

Solvent Blue 36
Formel: C₂₀H₂₂N₂O₂
Molekulargewicht: 322.41
Perfluoroktan: Siedepunkt: 105°C
Dampfdruck: 18,5 mbar bei 25°C

Aus dem semifluorierten Alkan und dem Farbstoff wurde durch Vermischen mit einem Flügelrührer eine homogene Lösung hergestellt, die anschließend mit dem perfluorierten Alkan homogen vermischt wurde. Die Dichte der Lösungen wurde bei Raumtemperatur (20°C) mittels Schwingkörper-Methode ermittelt. Verwendet wurde hierbei das Dichtemessgerät DA-100M der Firma Mettler-Toledo.

Die Zubereitung ließ sich einfach durch eine Spritze mit einer Kanüle von 25 Gauge aufziehen und daraus wieder entleeren. Sie war über eine Lagerzeit von 42 Stunden bei 120 °C stabil, was einer Lagerzeit bei Umgebungsbedingungen von etwa 3 Jahren und 8 Monaten entspricht (unter Berücksichtigung der van't-Hoff'schen Gleichung entspricht eine Reaktionsgeschwindigkeit von einer Stunde bei 100°C (Rückfluss einer wässrigen Lösung) etwa 32 Tagen bei 20 °C). Nach dieser Zeit zeigte die Lösung visuell unter dem Mikroskop keine Veränderung, also weder Separation noch Trübung. Ebenso ließ sich die Zubereitung ohne wesentliche visuell wahrnehmbare Veränderungen durch Sterilisation mittels Sterilfilter mit Porengröße 0,2 µm sterilisieren.

### Beispiel 2

| Farbstoff | Gehalt Semifluoriertes Alkan C₆F₁₃-C₈H₁₇ | Gehalt Perfluoriertes Alkan Perfluordecalin | Konzentration Farbstoff in g/L | Dichte [g/cm³] | Farbintensität |
|---|---|---|---|---|---|
| Solvent Blue 36 | 10 Vol.-% | 90 Vol.-% | 0,006 | 1,86 | zart |
| Solvent Blue 36 | 20 Vol.-% | 80 Vol.-% | 0,012 | 1,80 | leicht |
| Solvent Blue 36 | 30 Vol.-% | 70 Vol.-% | 0,018 | 1,73 | mittel |
| Solvent Blue 36 | 60 Vol.-% | 40 Vol.-% | 0,036 | 1,67 | stark |

| | | | | | |
|---|---|---|---|---|---|
| Perfluordecalin: Siedepunkt: 142°C Dampfdruck: 8 mbar bei 25°C | | | | | |

Die genannten Komponenten wurden wie in Beispiel 1 zu einer transparenten gefärbten Zubereitung formuliert. Die Dichte wurde wie in Beispiel 1 bestimmt.

Die Zubereitung ließ sich durch eine Spritze mit einer Kanüle von 25 Gauge aufziehen und daraus wieder entleeren. Sie war über eine Lagerzeit von 42 Stunden bei 120 °C stabil. Nach dieser Zeit zeigte die Lösung visuell unter dem Mikroskop keine Veränderung, also weder Separation noch Trübung. Die Zubereitung wurde mittels trockener Hitze bei einer Temperatur von 135°C 5 Stunden lang sterilisiert, wobei sie sich nicht visuell wahrnehmbar veränderte.

### Beispiel 3

| Farbstoff | Gehalt Semifluoriertes Alkan C₆F₁₃-C₈H₁₇ | Gehalt Perfluoriertes Alkan Perfluoroktan | Konzentration Farbstoff in g/L | Dichte [g/cm³] | Farbintensität |
|---|---|---|---|---|---|
| Solvent Blue 35 | 10 Vol.-% | 90 Vol.-% | 0,006 | 1,71 | keine |
| Solvent Blue 35 | 20 Vol.-% | 80 Vol.-% | 0,012 | 1,66 | leicht |
| Solvent Blue 35 | 30 Vol.-% | 70 Vol.-% | 0,018 | 1,62 | mittel |
| Solvent Blue 35 | 60 Vol.-% | 40 Vol.-% | 0,036 | 1,58 | stark |

### Farbstoff

### Solvent Blue 35

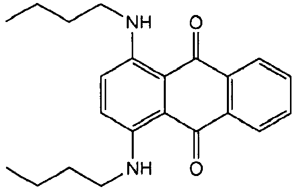

Aus dem semifluorierten Alkan und dem Farbstoff wurde durch Vermischen mit einem Flügelrührer eine homogene Lösung hergestellt, die anschließend mit dem perfluorierten Alkan homogen vermischt wurde. Die Dichte der Lösungen wurde bei Raumtemperatur (20 °C) mittels Schwingkörper-Methode ermittelt. Verwendet wurde hierbei das Dichtemessgerät DA-100M der Firma Mettler-Toledo.

Die Zubereitung ließ sich einfach durch eine Spritze mit einer Kanüle von 25 Gauge aufziehen und daraus wieder entleeren. Sie war über eine Lagerzeit von 42 Stunden bei 120 °C stabil, zeigte visuell unter dem Mikroskop keine Veränderung, also weder Separation noch Trübung. Ebenso ließ sich die Zubereitung ohne wesentliche visuell wahrnehmbare Veränderungen durch Sterilisation mittels Sterilfilter mit Porengröße 0,2 µm sterilisieren.

### Beispiel 4

| Farbstoff | Gehalt Semifluoriertes Alkan C₆F₁₃-C₈H₁₇ | Gehalt Perfluoriertes Alkan Perfluordecalin | Konzentration Farbstoff in g/L | Dichte [g/cm³] | Farbintensität |
|---|---|---|---|---|---|
| Solvent Blue 35 | 10 Vol.-% | 90 Vol.-% | 0,006 | 1,86 | zart |
| Solvent Blue 35 | 20 Vol.-% | 80 Vol.-% | 0,012 | 1,80 | leicht |
| Solvent Blue 35 | 30 Vol.-% | 70 Vol.-% | 0,018 | 1,73 | mittel |
| Solvent Blue 35 | 60 Vol.-% | 40 Vol.-% | 0,036 | 1,67 | stark |

Die genannten Komponenten wurden wie in Beispiel 1 zu einer transparenten gefärbten Zubereitung formuliert. Die Dichte wurde wie in Beispiel 1 bestimmt.

Die Zubereitung ließ sich durch eine Spritze mit einer Kanüle von 25 Gauge aufziehen und daraus wieder entleeren. Sie war über eine Lagerzeit von 42 Stunden bei 120 °C stabil. Nach dieser Zeit zeigte die Lösung visuell unter dem Mikroskop keine Veränderung, also weder Separation noch Trübung. Die Zubereitung wurde mittels trockener Hitze bei einer Temperatur von 135°C 5 Stunden lang sterilisiert, wobei sie sich nicht visuell wahrnehmbar veränderte.

### Beispiel 5

| Farbstoff | Gehalt Semifluoriertes Alkan C₆F₁₃-C₈H₁₇ | Gehalt Perfluoriertes Alkan Perfluoroktan | Konzentration Farbstoff in g/L | Dichte [g/cm³] | Farbintensität |
|---|---|---|---|---|---|
| Solvent Violet 13 | 10 Vol.-% | 90 Vol.-% | 0,006 | 1,71 | zart |
| Solvent Violet 13 | 20 Vol.-% | 80 Vol.-% | 0,012 | 1,66 | leicht |
| Solvent Violet 13 | 30 Vol.-% | 70 Vol.-% | 0,018 | 1,62 | mittel |
| Solvent Violet 13 | 60 Vol.-% | 40 Vol.-% | 0,036 | 1,58 | stark |

Aus dem semifluorierten Alkan und dem Farbstoff wurde durch Vermischen mit einem Flügelrührer eine homogene Lösung hergestellt, die anschließend mit dem perfluorierten Alkan homogen vermischt wurde. Die Dichte der Lösungen wurde bei Raumtemperatur (20 °C) mittels Schwingkörper-Methode ermittelt. Verwendet wurde hierbei das Dichtemessgerät DA-100M der Firma Mettler-Toledo.

Die Zubereitung ließ sich einfach durch eine Spritze mit einer Kanüle von 25 Gauge aufziehen und daraus wieder entleeren. Sie war über eine Lagerzeit von 42 Stunden bei 120 °C stabil, zeigte visuell unter dem Mikroskop keine Veränderung, also weder Separation noch Trübung. Ebenso ließ sich die Zubereitung ohne wesentliche visuell wahrnehmbare Veränderungen durch Sterilisation mittels Sterilfilter mit Porengröße 0,2 µm sterilisieren.

### Beispiel 6

| Farbstoff | Gehalt Semifluoriertes Alkan C₆F₁₃-C₈H₁₇ | Gehalt Perfluoriertes Alkan Perfluordecalin | Konzentration Farbstoff in g/L | Dichte [g/cm³] | Farbintensität |
|---|---|---|---|---|---|
| Solvent Violet 13 | 10 Vol.-% | 90 Vol.-% | 0,006 | 1,86 | zart |
| Solvent Violet 13 | 20 Vol.-% | 80 Vol.-% | 0,012 | 1,80 | leicht |
| Solvent Violet 13 | 30 Vol.-% | 70 Vol.-% | 0,018 | 1,73 | mittel |
| Solvent Violet 13 | 60 Vol.-% | 40 Vol.-% | 0,036 | 1,67 | stark |

Aus dem semifluorierten Alkan und dem Farbstoff wurde durch Vermischen mit einem Flügelrührer eine homogene Lösung hergestellt, die anschließend mit dem perfluorierten Alkan homogen vermischt wurde. Die Dichte der Lösungen wurde bei Raumtemperatur (20 °C) mittels Schwingkörper-Methode ermittelt. Verwendet wurde hierbei das Dichtemessgerät DA-100M der Firma Mettler-Toledo.

Die Zubereitung ließ sich einfach durch eine Spritze mit einer Kanüle von 25 Gauge aufziehen und daraus wieder entleeren. Sie war über eine Lagerzeit von 42 Stunden bei 120 °C stabil, zeigte visuell unter dem Mikroskop keine Veränderung, also weder Separation noch Trübung. Ebenso ließ sich die Zubereitung ohne wesentliche visuell wahrnehmbare Veränderungen durch Erhitzen auf 135 °C für 5 Stunden sterilisieren.

### Beispiel 7

| Farbstoff | Gehalt Semifluoriertes Alkan C₆F₁₃-C₈H₁₇ | Gehalt Perfluoriertes Alkan Perfluoroktan | Dichte [g/cm³] | Farbintensität |
|---|---|---|---|---|
| Solvent Green 5 | 30 Vol.-% | 70 Vol.-% | 1,62 | mittel |
| Solvent Green 5 | 60 Vol.-% | 40 Vol.-% | 1,58 | mittel |

### Farbstoff: Solvent Green 5 (Keyplast Yellow Green 7G)

### Diisobutyl 3,9-perylenedicarboxylate→ Fluoreszenzfarbstoff

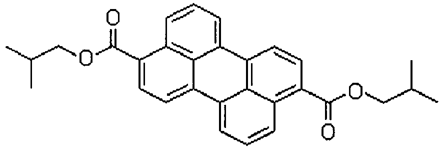

Formel: C₃₀H₂₈O₄ Molekulargewicht: 452.54

### Perfluoroktan F6H8

Es wurde eine Zubereitung mit dem fluoreszierenden Farbstoff Solvent Green 5 hergestellt. Dazu wurden die genannten Komponenten wie in Beispiel 1 zu einer transparenten gefärbten Zubereitung formuliert. Die Dichte wurde wie in Beispiel 1 bestimmt.

Die Zubereitung ließ sich durch eine Spritze mit einer Kanüle von 25 Gauge aufziehen und daraus wieder entleeren. Sie war über eine Lagerzeit von 42 Stunden bei 120 °C stabil. Nach dieser Zeit zeigte die Lösung visuell unter dem Mikroskop keine Veränderung, also weder Separation noch Trübung. Die Zubereitung konnte ohne wesentliche visuell wahrnehmbare Veränderungen durch Sterilisation mittels Sterilfilter mit Porengröße 0,2 µm sterilisiert werden.

### Beispiel 8

| Farbstoff | Gehalt Semifluoriertes Alkan C₆F₁₃-C₈H₁₇ | Gehalt Perfluoriertes Alkan Perfluordecalin | Dichte [g/cm³] | Farbintensität |
|---|---|---|---|---|
| Solvent Green 5 | 30 wt. % | 70 wt.% | 1,74 | mittel |
| Solvent Green 5 | 60 wt.% | 40 wt. % | 1,68 | mittel |

Es wurde eine Zubereitung mit dem fluoreszierenden Farbstoff Solvent Green 5 hergestellt. Dazu wurden die genannten Komponenten wie in Beispiel 1 zu einer transparenten gefärbten Zubereitung formuliert. Die Dichte wurde wie in Beispiel 1 bestimmt.

Die Zubereitung ließ sich durch eine Spritze mit einer Kanüle von 25 Gauge aufziehen und daraus wieder entleeren. Sie war über eine Lagerzeit von 42 Stunden bei 120 °C stabil. Nach dieser Zeit zeigte die Lösung visuell unter dem Mikroskop keine Veränderung, also weder Separation noch Trübung. Die Zubereitung wurde mittels trockener Hitze bei einer Temperatur von 135°C 5 Stunden lang sterilisiert, wobei sie sich nicht visuell wahrnehmbar veränderte.

### Beispiel 9

| Farbstoff | Gehalt Semifluoriertes Alkan C₄F₉-C₅H₁₁ | Gehalt Perfluoriertes Alkan Perfluordecalin | Konzentration Farbstoff in g/L | Dichte [g/cm³] | Farbintensität |
|---|---|---|---|---|---|
| Solvent Violet 13 | 10 Vol.-% | 90 Vol.-% | 0,006 | 1,86 | zart |

Es wurde eine Zubereitung mit dem Farbstoff Solvent Violet 13 hergestellt. Dazu wurden die genannten Komponenten wie in Beispiel 1 zu einer transparenten gefärbten Zubereitung formuliert. Die Dichte wurde wie in Beispiel 1 bestimmt.

### Beispiel 10

| Farbstoff | Gehalt Semifluoriertes Alkan C₆F₁₃-C₆H₁₃ | Gehalt Perfluoriertes Alkan Perfluordecalin | Konzentration Farbstoff in g/L | Dichte [g/cm³] | Farbintensität |
|---|---|---|---|---|---|
| Solvent Violet 13 | 10 Vol.-% | 90 Vol.-% | 0,006 | 1,87 | zart |

Es wurde eine Zubereitung mit dem Farbstoff Solvent Violet 13 hergestellt. Dazu wurden die genannten Komponenten wie in Beispiel 1 zu einer transparenten gefärbten Zubereitung formuliert. Die Dichte wurde wie in Beispiel 1 bestimmt.

## Patentansprüche

1. Zubereitung zur Verwendung in der Augenheilkunde oder Netzhautchirurgie enthaltend mindestens ein perfluoriertes Alkan, mindestens ein semifluoriertes Alkan und mindestens eine in dem semifluorierten Alkan gelöste farbgebende Substanz, wobei das semifluorierte Alkan ausgewählt ist aus:
R_{F}R_{H} oder R_{F}R_{H}R_{F}
wobei R_{F} eine lineare oder verzweigte Perfluoralkylgruppe mit 3 bis 20 Kohlenstoffatomen und R_{H} eine lineare oder verzweigte, gesättigte Alkylgruppe mit 1 bis 20 Kohlenstoffatomen ist, wobei die Gesamtkettenlänge 4 bis 30 Kohlenstoffatome beträgt und wobei das Verhältnis des perfluorierten Alkans zu dem semifluorierten Alkan, bezogen auf Volumen, 95:5 bis 5:95 beträgt.

2. Zubereitung zur Verwendung wie in Anspruch 1, **dadurch gekennzeichnet dass** die farbgebende Substanz ein Lösemittelfarbstoff oder Fettfarbstoff ist.

3. Zubereitung zur Verwendung wie in Anspruch 1 oder 2, **dadurch gekennzeichnet, dass** der Farbstoff ein Lösemittelfarbstoff ist, der ausgewählt ist aus der Gruppe bestehend aus: Solvent Blue 36, Solvent Blue 35, Solvent Green 5, Solvent Violet 13, Solvent Blue 8, Solvent Blue 18, Solvent Blue 63, Solvent Green 3, Solvent Green 7, Solvent Violet 10, Solvent Violet 12, Solvent Violet 26, Solvent Red 27, Solvent Yellow 56, Solvent Green 3, Solvent Yellow 33, Solvent Red 19, Solvent Red 1, Solvent Yellow 16.

4. Zubereitung zur Verwendung wie in Anspruch 1 oder 2, **dadurch gekennzeichnet, dass** der Farbstoff ein Fettfarbstoff ist, der ausgewählt ist aus der Gruppe bestehend aus: Sudan I, Sudan II, Sudan III, Sudan IV.

5. Zubereitung zur Verwendung wie in einem der vorhergehenden Ansprüche, **dadurch gekennzeichnet, dass** der Farbstoff fluoresziert, Licht im sichtbaren Wellenlängenbereich reflektiert oder durch Anregung mittels Energie zur Reflexion von Licht einer Wellenlänge im sichtbaren Wellenlängenbereich angeregt werden kann.

6. Zubereitung zur Verwendung wie in einem der vorhergehenden Ansprüche, **dadurch gekennzeichnet, dass** der Farbstoff in der fertigen Zubereitung in einer Menge von 0,08 bis 0,001 g/L, bevorzugter 0,07 bis 0,003 g/L, beispielsweise 0,06 g/L bis 0,006 g/L enthalten ist.

7. Zubereitung zur Verwendung wie in einem der vorhergehenden Ansprüche, **dadurch gekennzeichnet, dass** das Perfluoralkan ausgewählt ist aus der Gruppe bestehend aus: C₄F₁₀, C₅F₁₂, C₆F₁₄, C₇F₁₆, C₈F₁₈, C₉F₂₀ und C₁₀F₂₂.

8. Zubereitung zur Verwendung wie in einem der vorhergehenden Ansprüche, wobei die Zubereitung eine Dichte von mehr als 1 g/cm³ aufweist, wobei die Dichte bei Raumtemperatur von 20-25°C gemessen wird.

9. Zubereitung zur Verwendung wie in einem der vorhergehenden Ansprüche, **dadurch gekennzeichnet, dass** die Zubereitung eine Dichte von mehr als 1,33 g/cm³ und bevorzugt von mehr als 1,5 g/cm³ aufweist, wobei die Dichte bei Raumtemperatur von 20-25°C gemessen wird.

10. Zubereitung zur Verwendung wie in einem der vorhergehenden Ansprüche, **dadurch gekennzeichnet, dass** die Menge an semifluoriertem Alkan 5 bis 60 Vol.-%, bevorzugt 10 bis 40, und besonders bevorzugt 10 bis 20 Vol.-% bezogen auf das Gesamtvolumen der Zubereitung beträgt.

11. Zubereitung zur Verwendung wie in einem der vorhergehenden Ansprüche, weiterhin enthaltend einen arzneimittelwirksamen Bestandteil oder fettlösliche Antioxidans.

12. Zubereitung zur Verwendung wie in einem der vorhergehenden Ansprüche zur Verwendung in der Augenheilkunde oder Netzhautchirurgie, insbesondere für Vitrektomie oder chirurgische Eingriffe im Zusammenhang mit Netzhautablösung.

13. Zubereitung zur Verwendung wie in einem der Ansprüche 1 bis 10 zur Verwendung als Infusionsflüssigkeit in der Netzhautchirurgie.

14. Verfahren zur Herstellung einer Zubereitung nach einem der Ansprüche 1 bis 10, umfassend homogenes Vermischen der farbgebenden Substanz mit einem semifluorierten Alkan und Zugeben eines Perfluoroalkans.

15. Verfahren nach Anspruch 14, weiterhin umfassend Aufbewahren der Zubereitung und Filtrieren der Zubereitung vor Weiterverarbeitung.

## Claims

1. A preparation for use in ophthalmology or retinal surgery, containing at least one perfluorinated alkane, at least one semi-fluorinated alkane and at least one coloring substance dissolved in the semi-fluorinated alkane, wherein the semi-fluorinated alkane being selected from:
R_{F}R_{H} or R_{F}R_{H}R_{F}
wherein R_{F} is a linear or branched perfluoroalkyl group with 3 to 20 carbon atoms, and R_{H} is a linear or branched, saturated alkyl group with 1 to 20 carbon atoms, wherein the total chain length is from 4 to 30 carbon atoms, and wherein the ratio of the perfluorinated alkane to the semi-fluorinated alkane, relative to volume, is 95:5 to 5:95.

2. The preparation for the use according to claim 1, **characterized in that** the coloring substance is a solvent dye or fat dye.

3. The preparation for the use according to claim 1 or 2, **characterized in that** the dye is a solvent dye, which is selected from the group consisting of: Solvent Blue 36, Solvent Blue 35, Solvent Green 5, Solvent Violet 13, Solvent Blue 8, Solvent Blue 18, Solvent Blue 63, Solvent Green 3, Solvent Green 7, Solvent Violet 10, Solvent Violet 12, Solvent Violet 26, Solvent Red 27, Solvent Yellow 56, Solvent Green 3, Solvent Yellow 33, Solvent Red 19, Solvent Red 1, Solvent Yellow 16.

4. The preparation for the use according to claim 1 or 2, **characterized in that** the dye is a fat dye, selected from the group consisting of: Sudan I, Sudan II, Sudan III, Sudan IV.

5. The preparation for the use according to any one of the preceding claims, **characterized in that** the dye fluoresces, reflects light in the visible wavelength range or can be excited to reflect light having a wavelength within the visible wavelength range by means of excitation through energy.

6. The preparation for the use according to any one of the preceding claims, **characterized in that** the dye is contained in an amount of 0.08 to 0.001 g/L, preferably from 0.07 to 0.003 g/L, for example, 0.06 g/L to 0.006 g/L in the finished preparation.

7. The preparation for the use according to any one of the preceding claims, **characterized in that** the perfluoroalkane is selected from the group consisting of: C₄F₁₀, C₅F₁₂, C₆F₁₄, C₇F₁₆, C₈F₁₈, C₉F₂₀ and C₁₀F₂₂.

8. The preparation for the use according to any one of the preceding claims, **characterized in that** the preparation has a density of more than 1 g/cm³, wherein the density is measured at room temperature of 20-25°C.

9. The preparation for the use according to any one of the preceding claims, **characterized in that** the preparation has a density of more than 1,33 g/cm³, and preferably of more than 1.5 g/cm³, wherein the density is measured at room temperature of 20-25°C.

10. The preparation for the use according to one of the preceding claims, **characterized in that** the amount of semi-fluorinated alkane is 5 to 60 vol.-%, preferably 10 to 40, and most preferably 10 to 20 vol.-% relative to the total volume of the preparation.

11. The preparation for the use according to one of the preceding claims, further comprising a pharmaceutically active or fat-soluble compound.

12. The preparation for the use according to one of the preceding claims for use in ophthalmology or retinal surgery, especially for vitrectomy or surgical procedures in connection with retinal detachment.

13. The preparation for the use according to any one of claims 1 to 10 for use as infusion fluid in retinal surgery.

14. Method for preparing a composition according to any one of claims 1 to 10, comprising homogeneously mixing the coloring substance with a semi-fluorinated alkane and adding of a perfluoroalkane.

15. The method of claim 14, further comprising storing the preparation and filtrating of the preparation before further processing.

## Revendications

1. Préparation pour l'utilisation dans l'ophtalmologie ou la chirurgie rétinienne, contenant au moins un alcane perfluoré, au moins un alcane semi-fluoré et au moins une substance colorante dissoute dans l'alcane semi-fluoré, ledit alcane semi-fluoré étant choisi parmi :
R_{F}R_{H} ou R_{F}R_{H}R_{F}
où R_{F} est un groupe perfluoroalkyle linéaire ou ramifié ayant 3 à 20 atomes de carbone, et
R_{H} est un groupe alkyle saturé, linéaire ou ramifié, ayant 1 à 20 atomes de carbone, la longueur totale de chaîne étant comprise entre 4 et 30 atomes de carbone,
dans laquelle le rapport de l'alcane perfluoré à l'alcane semi-fluoré, par rapport au volume, est compris entre 95 : 5 et 5 : 95.

2. Préparation pour l'utilisation selon la revendication 1, **caractérisée par le fait que** ladite substance colorante est un colorant soluble dans les solvants ou une substance lipochrome.

3. Préparation pour l'utilisation selon la revendication 1 ou 2, **caractérisée par le fait que** le colorant est un colorant soluble dans les solvants qui est choisi dans le groupe constitué par: le Solvent Blue 36, le Solvent Blue 35, le Solvent Green 5, le Solvent Violet 13, le Solvent Blue 8, le Solvent Blue 18, le Solvent Blue 63, le Solvent Green 3, le Solvent Green 7, le Solvent Violet 10, le Solvent Violet 12, le Solvent Violet 26, le Solvent Red 27, le Solvent Yellow 56, le Solvent Green 3, le Solvent Yellow 33, le Solvent Red 19, le Solvent Red 1, le Solvent Yellow 16.

4. Préparation pour l'utilisation selon la revendication 1 ou 2, **caractérisée par le fait que** le colorant est une substance lipochrome qui est choisie dans le groupe constitué par : le Sudan I, le Sudan II, le Sudan III, le Sudan IV.

5. Préparation pour l'utilisation selon l'une quelconque des revendications précédentes, **caractérisée par le fait que** le colorant est fluorescent, réfléchit la lumière dans le domaine des longueurs d'onde visibles ou peut être incité, par une excitation au moyen d'énergie, à réfléchir de la lumière d'une longueur d'onde dans le domaine des longueurs d'onde visibles.

6. Préparation pour l'utilisation selon l'une quelconque des revendications précédentes, **caractérisée par le fait que** le colorant est contenu dans la préparation finie en une quantité comprise entre 0,08 et 0,001 g/L, de manière plus préférée entre 0,07 et 0,003 g/L, par exemple entre 0,06 g/L et 0,006 g/L.

7. Préparation pour l'utilisation selon l'une quelconque des revendications précédentes, **caractérisée par le fait que** le perfluoroalcane est choisi dans le groupe constitué par : C₄F₁₀, C₅F₁₂, C₆F₁₄, C₇F₁₆, C₈F₁₈, C₉F₂₀ et C₁₀F₂₂.

8. Préparation pour l'utilisation selon l'une quelconque des revendications précédentes, **caractérisée par le fait que** la préparation présente une densité supérieure à 1 g/cm³, la densité étant mesurée à la température ambiante de 20 à 25 °C.

9. Préparation pour l'utilisation selon l'une quelconque des revendications précédentes, **caractérisée par le fait que** la préparation présente une densité supérieure à 1,33 g/cm³, et de préférence supérieure à 1,5 g/cm³, la densité étant mesurée à la température ambiante de 20 à 25 °C.

10. Préparation pour l'utilisation selon l'une quelconque des revendications précédentes, **caractérisée par le fait que** la quantité d'alcane semi-fluoré est comprise entre 5 et 60 % volumétrique, de préférence entre 10 et 40, et d'une manière particulièrement préférée entre 10 et 20 % volumétrique par rapport au volume total de la préparation.

11. Préparation pour l'utilisation selon l'une quelconque des revendications précédentes, contenant en outre un composant à efficacité médicamenteuse ou liposoluble.

12. Préparation pour l'utilisation selon l'une quelconque des revendications précédentes, pour l'utilisation dans l'ophtalmologie ou la chirurgie rétinienne, en particulier pour la vitrectomie ou des interventions chirurgicales en relation avec le décollement de la rétine.

13. Préparation pour l'utilisation selon l'une quelconque des revendications 1 à 10, pour l'utilisation en tant que liquide pour perfusion dans la chirurgie rétinienne.

14. Procédé de production d'une préparation selon l'une quelconque des revendications 1 à 10, comprenant le mélange homogène de la substance colorante à un alcane semi-fluoré et l'ajout d'un perfluoroalcane.

15. Procédé selon la revendication 14, comprenant en outre la conservation de la préparation et la filtration de la préparation avant le traitement ultérieur.
